# EUROPÄISCHE PATENTANMELDUNG

(11) **EP 0 647 707 A2**
(43) Veröffentlichungstag der Anmeldung: **12.04.1995**
(21) Anmeldenummer: 94890117.8
(22) Anmeldetag: 07.07.1994
(51) Int. Cl.: C12M 1/00, B01L 7/00

(54) **Thermokammer**

(30) Priorität: 20.08.1993 AT 1684/93
(71) Anmelder: SY-LAB Vertriebsges.m.b.H., A-3002 Purkersdorf (AT)
(72) Erfinder: Futschik, Karl, Dr., A-2344 Maria Enzersdorf (AT); Schiessl, Roland, A-1170 Wien (AT); Pfützner, Helmut, Prof. Dr., A-5640 Bad Gastein (AT); Fränzl, Gert, A-3002 Purkersdorf (AT)
(74) Vertreter: Müllner, Erwin, Dr.

(57) **Zusammenfassung**

Eine Thermokammer zur Temperaturkonstanthaltung von biologischen Proben in Meßgefäßen (13) umfaßt Seitenwände (1, 2, 3, 4), eine Boden platte (5) und gegebenenfalls einen Deckel (27). Mindestens eine der Seitenwände (1,2) ist thermisch leitend und direkt oder indirekt kühl- und bzw. oder beheizbar. Im Inneren sind thermische leitfähige Stege wie etwa Platten (8, 9, 10, 11, 12, 15, 16, 17), z.B. mit gewellter Oberfläche, vorgesehen, die mit der oder den beheizten oder gekühlten Seitenwänden (1, 2) der Thermokammer in inniger Berührung stehen. Die Stege bzw. Platten (8, 9, 10, 11, 12, 14, 15, 16, 17, 18) sind im Abstand zueinander angeordnet und schließen Meßgefäße (13) ein, welche durch den Spalt zwischen den einzelnen Platten (8, 9, 10, 11, 12, 14,15, 16, 17, 18) für Messungen und für Bestrahlung (Belichtung) zugänglich sind. Die Platten (8, 9, 10, 11, 12, 14 15, 16, 17, 18) haben Oberflächen mit parallelen Erzeugenden, die etwa einer Sinuslinie folgen. Die Sinuslinien der beiden Oberflächen einer Platte können gleichphasig oder gegenphasig laufen. In den konkaven Zonen der Platten werden die Meßgefäße (13) gehalten.

## Beschreibung

Die Erfindung betrifft eine Thermokammer mit mindestens einer thermisch leitfähigen Wand zur Temperierung bzw. zur Temperaturkonstanthaltung einer Mehrzahl von biologischen Proben in Meßgefäßen, beispielsweise zur Registrierung des Wachstums von Mikroorganismen, z.B. von Bakterien.

Um ein stabiles Klima für das Wachstum von Bakterien in Meßgefäßen zu schaffen, werden entweder Thermokammern oder Thermoblöcke verwendet. Während Thermokammern eine Zone mehrseitig oder allseitig begrenzen, in welche die Proben bzw.Meßgefäße eingebracht, beobachtet und analysiert werden können, sind Thermoblöcke kompakte wärmeleitfähige Massen, die Ausnehmungen aufweisen, welche die Meßgefäße zur Wärmeleitung eng umschließen. Damit liefern sie homogene thermische Verhältnisse, haben anderseits aber den Nachteil, daß die Gefäßwände - für Zwecke der Manipulation oder Messung - nicht zugänglich sind, und auch den Nachteil hoher Masse.

Die Messungen des Bakterienwachstums erfolgen beispielsweise elektrisch über die Impedanz des Gefäßinhaltes zwischen zwei oder mehreren Elektroden, wobei auch der Trübungsgrad bestimmt wird und eine Bestrahlung, sei es durch sichtbares Licht oder unsichtbare Wellen, auch durch elektrische oder magnetische Felder bzw. radioaktive Medien vorgesehen sein kann. Aufzeichnungen über die äußeren Parameter und die Meßwerte während eines Beobachtungszeitraumes werden automatisch durchgeführt. Der Versuchsablauf ist programmgesteuert.

Die Erfindung bezieht sich auf die Kategorie der Thermokammern und zielt darauf ab, im Inneren eine gleichmäßige Temperaturverteilung zu schaffen und gleichzeitig eine optische Überwachung bzw. Strahlungsbeeinflussung der Proben zu ermöglichen. Dies wird dadurch erreicht, daß das Innere der Thermokammer thermisch leitfähige Stege oder Platten zur Homogenisierung der Temperaturverteilung aufweist, die an zwei insbesondere parallel zueinander liegenden Wänden der Thermokammer, von denen wenigstens eine Wand (1 oder 2) leitfähig ist, zur Wärmeleitung anliegen und zueinander einen wenigstens in Teilbereichen gleichen oder größeren Abstand als ein Maß des Querschnittes der Meßgefäße, insbesondere des Durchmessers bei zylindrischen Meßgefäßen, aufweisen. Die Stege oder Platten, welche selbständige Baukörper bilden, übernehmen die Wärmeleitung von der oder den beheizten oder gekühlten leitfähigen Wänden der Thermokammer in den Innenraum, wobei die Steg- oder Plattenoberflächen bezüglich der Wärmestrahlung spezifisch behandelt werden können. Seitlich wird ein Wärmeübergang etwa durch großflächige, innige Berührung der Stege oder Platten mit den Wänden geschaffen. Es sind mehrere Stege oder Platten in der Kammer vorgesehen, die Unterteilungen für die einzubringenden Meßgefäße schaffen. Dadurch erfolgt eine thermische Entkoppelung der Meßgefäße. Eine Beeinflussung benachbarter Meßgefäße wird bei individueller Manipulation wie etwa Austausch eines Meßgefäßes, in engen Grenzen gehalten. Die Stege oder Platten können neben der Aufgabe der Wärmeleitung auch Haltefunktion für die Meßgefäße erfüllen, wenn Linienberührung oder Flächenberührung mit diesen erfolgt. Es ist zweckmäßig, wenn eine Mehrzahl von Platten, insbesondere aus Aluminium, vorgesehen ist, die parallel zueinander zwischen thermisch leitfähigen Wänden der Thermokammer gehalten sind. Die Platten können an den Enden rechtwinkelig umgebogen bzw. abgewinkelt sein und mit diesen abgewinkelten Schenkeln an den geheizten oder gekühlten Wänden der Thermokammer anliegen und den Wärmeübergang auf die Platten durchführen. Bei einer anderen Ausführungsform weisen die Platten Flachseiten auf, deren Erzeugenden etwa einer Sinuslinie folgen, wobei die Sinuslinien der Flachseiten einer Platte gleichphasig oder um 180° phasenverschoben zueinander verlaufen. Die äußere Form dieser Platten entspricht entweder jener eines Wellbleches, wobei die Wandstärken größer, also z.B. 8mm, sind oder aber es ändert sich die Wandstärke der Platten periodisch, z,B. von20 mm auf 8mm, wodurch sich an den Seitenwänden einander gegenüberliegende rinnenartige Ausnehmungen ergeben. Es ist zweckmäßig, wenn die Sinuslinien einander unmittelbar gegenüberliegender Flachseiten benachbarter Platten um 180° zueinander versetzt sind. Auf diese Weise ergeben sich wärmeleitfähige Einbauten in der Thermokammer, zwischen welchen die Meßgefäße einen Halt finden. Für eine Flächenberührung zwischen den Platten und üblichen kreiszylindrischen Meßge - fäßen sieht eine besondere Ausführungsform vor, daß wenigstens die konkave Halbwelle der Sinuslinie einer Platte annähernd einem Kreisbogen folgt. Die Einbuchtungen der Platten können so dem Kreisquerschnitt der Meßgefäße besser angepaßt werden. Eine andere Ausführungsform ist dadurch gekennzeichnet, daß die Platten an ihren Flachseiten, die als Ebenen ausgebildet sind, parallel zueinander rinnenförmige Ausnehmungen, beispielsweise mit etwa halbkreisförmigen oder V-förmigen Querschnitt aufweisen und daß die Platten parallel zueinander unter Bildung eines Spaltes, aneinanderreihbar sind, wobei die Ausnehmungen als Halterung für die Meßgefäße einander paarweise spiegelbildlich gegenüberliegen. Hier wird von einer welligen Außenfläche der Platten Abstand genommen und es sind die Nuten oder Einschnitte im Abstand zueinander vorgesehen, wobei die Flächen zwischen den Nuten oder Einschnitten Ebenen darstellen.

Besonders zweckmäßig ist es, wenn die Stege bzw. Platten in einen Rahmen, beispielsweise mit Schienen, einschiebbar sind, der die Position der Platten zueinander festlegt und fixiert. Der Rahmen kann Teil der thermisch leitfähigen Wand der Thermokammer sein oder mit diesem in wärmeleitfähiger Verbindung stehen, sodaß z.B. über Schienen, in welche die Platten einschiebbar sind, die Wärmeübertragung mit geringen Übergangsverlusten stattfinden kann. Ein Stecksystem gestattet das rasche Auswechseln der Platten etwa gegen solche mit anderen Querschnittsformen oder Wandstärken. Der Rahmen kann ferner elektrische Stromzuführungen für individuelle Heiz- oder Kühleinrichtungen (z.B. nach dem Peltiereffekt) aufweisen. Diese sind besonders bei großen Thermokammern oder bei häufigem Wechsel der Meßgefäße sowie auch für zonenweise unterschiedliche Bedingungen innerhalb der Thermokammer zweckmäßig. Es kann auch in den zwischen den Stegen oder Platten gebildeten Spalt Kühlluft oder Heißluft einblasbar sein. Bei einer sehr praxisgerechten Ausführung der Thermokammer sind in den durch den Abstand der Stege oder Platten gebildeten Spalt optische Meßeinrichtungen, z.B. Sensoren zur Trübungsmessung im Durchlicht- oder Reflexionsverfahrens einschiebbar oder es erfolgt eine Belichtung, Bestrahlung oder eine Beaufschlagung durch ein magnetisches oder elektrisches Feld. Dazu können in den Spalt z.B. Printplatten mit elektrischen Komponenten, Lichtleitersysteme aber auch Luftschläuche etwa zur Probenbegasung eingebracht werden. Ebenso können mechanische Komponenten eingebracht werden, die eine Rüttelbewegung der Gefäße bewirken bzw. magnetische Komponenten zur Anregung von in den Gefäßen untergebrachten Magnetrührern oder -partikeln. Unter den genannten Beaufschlagungen zeigen die Proben spezielle Veränderungen (z.B. beschleunigtes Mikroorganismenwachstum), die sich in Meßergebnissen niederschlagen und zu wertvollen Erkenntnisse führen können. Da die Impedanzmessung zwischen den Elektroden zu signifikanten Werten führt, wenn verschiedene Umweltbedingungen vorliegen, ist es zweckmäßig, wenn die Elektroden, wie bekannt, zur Impedanzmessung vorgesehen sind und eine Strahlungsquelle, z.B. Lichtquelle, im Spalt zu Vergleichsmessungen ein- und ausschaltbar ist.

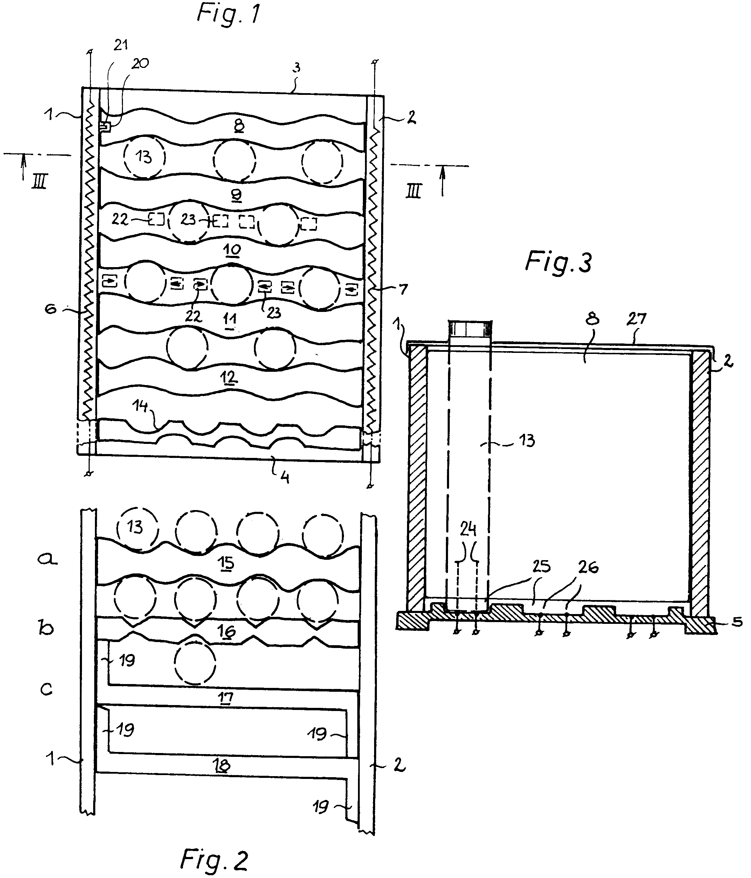

Ausführungsbeispiele des Erfindungsgegenstandes sind in den Zeichungen schematisch dargestellt. Fig. 1 zeigt einen Teil einer Thermokammer von oben, Fig. 2 a, b und c Varianten für Stege bzw. Platten und Fig. 3 einen Querschnitt nach der Linie III-III in Fig. 1.

Gemäß Fig. 1 und 3 ist eine Thermokammer als Behälter mit vier paarweise parallelen Seitenwänden 1, 2, 3, 4 und mit einer Bodenplatte 5 ausgebildet. Zwei der Seitenwände 1, 2 sind im Ausführungsbeispiel mit Heiz- und bzw. oder Kühlschlangen 6, 7 bzw. Matten ausgestattet. Elektrische Widerstandsheizung bzw. Kühlung nach dem Lindeverfahren oder mit Hilfe des Peltiereffektes ist möglich. Eine thermische Isolation, die die Thermokammer allseitig umgibt, wurde zur besseren Übersichtlichkeit in den Zeichnungen weggelassen. Fig. 1 zeigt, daß die Thermokammer beliebige Länge aufweisen kann.

Im Inneren der Thermokammer sind thermisch leitfähige Stege in Form von Platten 8, 9 10, 11, 12 vorgesehen, welche die Wände 1 und 2 verbinden und parallel zu den Wänden 3 und 4 liegen. Um die thermischen Übergangsverluste zwischen den Wänden 1 bzw. 2 und den Platten 8, 9, 10, 11 möglichst klein zu halten, sind die Berührungsflächen sehr groß ausgeführt und eine enge Passung ist vorgesehen. Bei einer konkreten Ausführungsform werden die Seitenwände 1, 2 gegen die Platten 8, 9, 10, 11, 12 z.B. mit Zugschrauben vorgespannt. Die Seitenwände der Platten sind annähernd sinusförmig gewellt, wobei die Erzeugenden parallel zueinander liegen und der Sinuslinie beiderseits jeder Platte folgen. Die Sinuslinieneiner jeden Platte 8 bzw. 9, 10, 11, 12 laufen gleichphasig, parallel zueinander. Die Sinuslinien von benachbarten Platten (z.B. 8 und 9) an einander gegenüberliegenden Seitenwänden sind in Gegenphase, sodaß konkave Plattenbereiche einander gegenüberliegen und jeweils ein zylindrisches Kulturgefäß 13 halten. Hier sind die Platten 8 bis 12 mit sehr flacher Wellung dargestellt. Es ist aber möglich, die Wellung wesentlich ausgeprägter auszuführen, sodaß zwischen Platten und Meßgefäßen eine Flächenberührung, z.B. in einem Winkel von 90° erfolgt. Die Platten können z.B. aus Aluminium durch spanabhebende Fertigung oder aus einer Legierung im Gußverfahren hergestellt werden.

In Fig. 1 ist noch als Variante eine Platte 14 dargestellt, die planparallele Seitenwände mit rinnenförmigen Ausnehmungen (Kreiszylinderflächen) aufweist. Eine solche Platte ist besonders leicht herstellbar.

Fig. 2 a, b, c zeigt weitere Varianten für Platten 15, 16, 17 und 18. Die Platte 15 weist gewellte Seitenwände auf, wobei die Wellungen (Sinuslinien) in Gegenphase liegen. Wenn solche Platten 15 in der Thermokammer parallel zueinander angeordnet werden, dann liegen die Meßgefäße 13 jeweils in Zeilen und Spalten (Fig. 2) und nicht versetzt, wie dies in Fig. 1 dargestellt ist. Ähnliches gilt auch für eine Platte 16, die V-förmige Rillen zur Halterung der Meßgefäße 13 trägt.

Die Platten 17, 18 haben ebene Seitenwände mit abgewinkelten Schenkeln 19 an den Enden, die einen großflächigen Wärmeübergang von den Seitenwänden 1, 2 ermöglichen. Die Schenkel 19 können dicht aneinandergereiht werden, sodaß sich die Abstände der Platten 17, 18 von selbst einstellen. Die Wände 1, 2 werden gegen die Platten gespannt.

In Fig. 1 ist zur Positionierung der Platten als Beispiel bei Platte 8 eine Nut 20 dargestellt, die auf eine Schiene 21 aufgeschoben werden kann. Derartige Schienen (z.B. U-Schienen) können die Platten 8-12 sowie 15, 16 auch seitlich umgreifen. Zwischen jeweils zwei Platten 8-12 und 15, 16 liegt jeweils ein Spalt, der einen Zugang zu den Meßgefäßen 13 von der Seite ermöglicht um etwa eine Belichtung oder eine optische Messung durchzuführen. In Fig. 1 und 3 sind Sensoren 22, 23 für derartige Messungen dargestellt.

Die Platten dienen der Wärmeleitung in das Innere der Thermokammer und schaffen eine homogene Temperaturverteilung sowie diskrete Klimazonen, sodaß eine Entkoppelung der Kulturgefäße 13 möglich ist. Zusatzheizungen oder Kühlungen in den Platten selbst ermöglichen lokale Beeinflussungen, z.B. bei Störungen durch Entnahme eines Meßgefäßes.

Weitere Messungen können, wie bekannt, auf elektrischem Wege, z.B. durch Impedanzmessung zwischen zwei Elektroden 24, durchgeführt werden. Dazu weist die Thermokammer eine Bodenplatte 5 mit Ausnehmungen 25 zur Zentrierung der Meßgefäße 13 auf. In diese Ausnehmungen 25 werden die Meßgefäße 13 hineingestellt. Ringkontakte 26 stellen die Verbindung zu den Elektroden im Inneren der Gefäße 13 her. Die Thermokammer ist nach oben hin durch einen Deckel 27 abgeschlossen.

Die Thermokammer ist im Baukastensystem vergrößerbar und kann durch Austausch der Platten an Gefäße beliebiger Größe angepaßt werden. Auch können zur Messung im Spalt zwischen den Platten Printplatten, z.B. nahe den Wänden 1, 2, in den Spalt eingeschoben werden, die z.B. die Bauelemente für eine Belichtung auf einer Seite der Gefäße und eine Lichtmessung anderseits an anderen Enden des Spaltes enthalten. Ferner ist es auch möglich, jedem oder einigen der Meßgefäße individuell Licht- oder Strahlenerzeuger und bzw. oder Meßeinrichtungen im Spalt zuzuordnen. Es können dazu Lichtleiter verwendete werden. Ein Umrüsten auf geänderte thermische Eigenschaften der Thermokammer ist sehr rasch möglich. Damit wird etwa die Lagerhaltung beim Hersteller positiv beeinflußt.

## Patentansprüche

1. Thermokammer mit mindestens einer thermisch leitfähigen Wand zur Temperierung bzw. zur Temperaturkonstanthaltung einer Mehrzahl von biologischen Proben in Meßgefäßen, beispielsweise zur Registrierung des Wachstums von Mikroorganismen, zB. von Bakterien, dadurch gekennzeichnet, daß das Innere der Thermokammer thermisch leitfähige Stege oder Platten (8,9, 10, 11, 12, 14,15,16,17,18) zur Homogenisierung der Temperaturverteilung aufweist, die an zwei insbesondere parallel zueinander liegenden Wänden (1,2) der Thermokammer, von denen wenigstens eine Wand (1 oder 2) leitfähig ist, zur Wärmeleitung anliegen und zueinander einen wenigstens in Teilbereichen gleichen oder größeren Abstand als ein Maß des Querschnittes der Meßgefäße (13), insbesondere des Durchmessers bei zylindrischen Meßgefäßen, aufweisen.

2. Thermokammer nach Anspruch 1, dadurch gekennzeichnet, daß eine Mehrzahl von Platten (8, 9, 10, 11, 12, 14, 15, 16, 17, 18), insbesondere aus Aluminium, vorgesehen ist, die parallel zueinander zwischen zwei leitfähigen Wänden (1, 2) der Thermokammer gehalten sind.

3. Thermokammer nach Anspruch 2, dadurch gekennzeichnet, daß die Platten (8,9, 10, 11, 12, 15) Flachseiten aufweisen, deren Erzeugenden etwa einer Sinuslinie folgen, wobei die Sinuslinien der Flachseiten einer Platte (8, 9, 10, 11, 12, 15) gleichphasig oder um 180° phasenverschoben zueinander verlaufen.

4. Thermokammer nach Anspruch 3, dadurch gekennzeichnet, daß die Sinuslinien einander unmittelbar gegenüberliegender Flachseiten benachbarter Platten (8, 9, 10, 11, 12, 14,15 16, 17, 18) um 180° zueinander versetzt sind.

5. Thermokammer nach den Ansprüchen 3 und 4, dadurch gekennzeichnet, daß wenigstens die konkave Halbwelle der Sinuslinie einer Platte (14) annähernd einem Kreisbogen folgt.

6. Thermokammer nach den Ansprüchen 1 ud 2, dadurch gekennzeichnet, daß die Platten (14, 16) an ihren Flachseiten, die als Ebenen ausgebildet sind, parallel zueinander rinnenförmigen oder V-förmigen Querschnitt aufweisen und daß die Platten (14, 16) parallel zueinder unter Bildung eines Spaltes, aneinanderreihbar sind, wobei die Ausnehmungen als Halterung für die Meßgefäße (13) einander paarweise spiegelbildlich gegenüberliegen.

7. Thermokammer nach einem der Ansprüche 1 bis 6, dadurch gekennzeichnet, daß die Stege bzw. Platten (8, 9, 10, 11, 12, 14, 15, 16, 17, 18) in einen Rahmen, beispielsweise mit Schienen (21), einschiebbar sind, der die Position der Platten zueinander festlegt und fixiert.

8. Thermokammer nach Anspruch 7, dadurch gekennzeichnet, daß der Rahmen elektrische Stromzuführungen für individuelle Heiz- oder Kühleinrichtungen jeder Platte (8, 9, 10, 11, 12 14, 15, 16, 17, 18) aufweist.

9. Thermokammer nach einem der Ansprüche 1 bis 8, dadurch gekennzeichnet, daß in den zwischen den Stegen oder Platten (8, 9,, 10, 11, 12,14, 15, 16, 17,18) gebildeten Spalt Kühlluft oder Heißluft einblasbar ist.

10. Thermokammer nach einem der Ansprüche 1 bis 9, dadurch gekennzeichnet, daß in den durch den Abstand der Stege oder Platten gebildeten Spalt elektrische, optische, magnetische oder mechanische Meßeinrichtungen, z.B. Sensoren (22, 23) zur Trübungsmessung im Durchlicht- oder Reflexionsverfahren einschiebbar sind oder daß eine Belichtung, Bestrahlung oder eine Beaufschlagung durch ein magnetisches oder elektrisches Feld erfolgt.

11. Thermokammer nach einem der Ansprüche 1 bis 10, dadurch gekennzeichnet, daß im Inneren der Therokammer unterhalb der Stege eine Bodenplatte (5) vorgesehen ist, die Kontakte (26) aufweist, welche elektrische Stromkreise zu Sonden bzw.Elektroden (24) in den Meßgefäßen (13) schließen.

12. Thermokammer nach Anspruch 11, dadurch gekennzeichnet, daß die Elektroden (24), wie bekannt, zur Impedanzmessung vorgesehen und eine Strahlungsquelle, z.B. Lichtquelle, im Spalt zu Vergleichsmessungen ein- und ausschaltbar ist.
